Europäisches Patentamt

European Patent Office

Office européen des brevets

(11) Publication number: **0 337 617**
**A2**

# EUROPEAN PATENT APPLICATION

(21) Application number: **89302774.8**

(22) Date of filing: **21.03.89**

(51) Int. Cl.⁴: **A61B 17/42 , A61B 10/00 , A61D 1/08 , A61D 7/02 , F16K 15/14**

(30) Priority: **12.04.88 GB 8808572**

(43) Date of publication of application:
**18.10.89 Bulletin 89/42**

(84) Designated Contracting States:
**AT BE CH DE ES FR GR IT LI LU NL SE**

(71) Applicant: **H.G. Wallace Limited**
**Whitehall Road**
**Colchester Essex CO2 8JH(GB)**

(72) Inventor: **Wallace, Henry George**
**Whitehall Road**
**Colchester Essex CO2 8JH(GB)**

(74) Representative: **Stebbing, Peter John Hunter**
**Riches, Mill Lane**
**Bradfield Manningtree Essex CO11 2QP(GB)**

(54) **Pressure actuated valve particularly for biological use.**

(57) The present invention provides a single channel needle assembly particularly for the recovery of a biologically active component such as a human embryo, which assembly comprises a single channel transcutaneous needle (1),
a three-way connector assembly (2) operatively associated with a remote end of said needle; wherein one channel (4) of said three-way assembly (2) is obturated by a valve assembly (5) comprising a resilient membrane (10) provided with at least one slit (6.7.) held under tension across said channel, said resilient membrane being arranged such that the slit remains closed during normal aspiration.
The invention also provides a method for the recovery of a biologically active component utilising the single channel needle assembly as described.

EP 0 337 617 A2

# PRESSURE ACTUATED VALVE PARTICULARLY FOR BIOLOGICAL USE

The present invention relates to a pressure actuated valve particularly for biological use.

Pressure actuated valves are known in the art. Such valves are adapted to open when a predetermined pressure differential has been established, and to close when such differential is removed, see for e.g. US-A-3734126. With delicate biological materials, however, the moving parts associated with such valve assemblies render them quite unsuited for use since the protrusions and associated parts tend to damage biological material; particularly sensitive biological material such as oocytes. According, therefore, to an aspect of the invention there is provided a pressure actuated valve characterised by a resilient membrane having at least one slit and held across a fluid pathway under tension, whereby a pressure above a threshold value is required to open the slit.

The pressure may be positive or negative and may be exerted upstream or downstream of the valve so as to exceed the threshold value. Where such pressure differentials are exerted it is possible to arrange that only one of the positive or negative pressures actuate the valve. This may be effected by arranging that the slit portions of the membrane are not merely planar, but overlap at a pre-determined side.

The membrane may have a single or multiple slit therein and may be formed of one or a plurality of layers, which layers may be conveniently utilised to form the overlapping embodiment. By utilising overlapping slits a one-way facility can be provided according to the sides the overlapping slits are applied and the direction, upstream or downstream, from which the pressure is applied.

In a particular embodiment the valves of the invention are applied to a biologically active component, e.g. an oocyte, recovery device. Accordingly, there is provided a single channel needle assembly for the recovery of a biologically active component, which assembly comprises:
a single channel transcutaneous needle,
a three-way connector assembly operatively associated with a remote end of said needle;
characterised in that one channel of said three-way assembly is obturated by a valve assembly comprising a resilient membrane provided with at least one slit held under tension across said one channel, said resilient membrane bring arranged such that the slit remains closed during normal aspiration. In such an arrangement the three-way connector is preferably connected between the remote end of the needle and a collection channel leading to a collection vessel. The third channel is a flushing channel provided with a membrane valve as just described. By this means the pressure from a hypodermic syringe, for example, is sufficient to deform the membrane to allow the flushing solution to enter the needle. When flushing has been completed a negative pressure may be applied to aspirate the collection channel which draws a biological component into a collecting vessel via the collecting channel, without emptying the flushing channel because this is closed by the membrane valve. It is most desirable that the valve is flushed with the sides of the main channel in the three-way connector so that no protruburances which might damage the oocyte are present. By this means the oocytes can be withdrawn past the entrance to the flushing channel without bruising and without the possibility that they will be taken up into the flushing channel.

In another aspect of the invention there is provided a method for the recovery of a biologically active component, which method comprises;
inserting a single channel needle assembly into the body to a site juxtaposed said component,
introducing a flushing solution into the body via said needle, and aspirating said component via said single channel;
characterised in that the remote end of the needle is operatively associated with a three-way valve assembly, said valve assembly being provided in one channel with a resilient membrane provided with at least one slit held under tension across said one channel, said resilient membrane being arranged such that said slit remains closed during normal aspiration, but opens under pressure from said flushing solution.

In a final aspect of the invention there is provided a method of inducing pregnancy in an infertile mammalian female, which method comprises recovering an oocyte from the follicle by inserting a single channel needle assembly into the ovarian follicle, introducing a flushing solution into the follicle via said needle, aspirating said component via said single channel, causing said oocytes to be fertilised in vitro and implanting said fertilised oocytes into the uterine endometrium to establish pregnancy,
characterised in that the recovery of the oocytes is effected by an arrangement wherein the remote end of the needle is operatively associated with a three-way valve assembly, said valve assembly being provided in one channel with a resilient membrane provided with at least one slit held under tension across said one channel, said resilient membrane being arranged such that said slit remains closed during normal aspiration, but opens under pressure from said flushing solution in use.

The invention will now be described, by way of illustration only, with reference to the accompanying drawings wherein:

Figure 1 shows in vertical cross-section a three-way collector incorporating a valve assembly in accordance with the present invention, and

Figures 2 and 3 show plan views from above of valve membranes in accordance with the present invention.

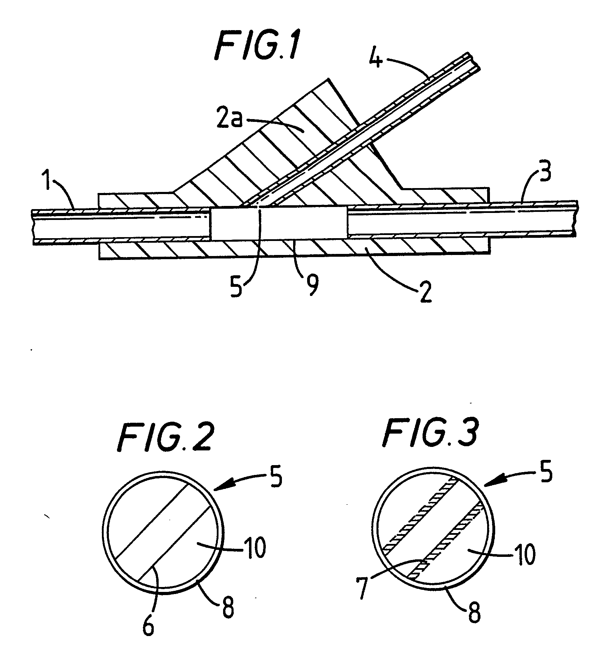

In accordance with the present invention, and with reference to Figure 1, a three-way connector 2 formed of a trans parent plastics material is provided with a longitudinal channel 9. Said channel 9 is a sliding fit relative to the one end of a transcutaneous single lumen needle 1, and in respect of a collecting channel catheter 3. As an initial point of assembly the needle 1 and the channel 3 are urged into sliding abutment with the body of the three-way connector 2.

The body of the three-way connector 2 is provided with an upstanding triangular portion 2a which in turn supports a flushing channel 4 which passes through the body of the support 2a and terminates between the respective ends of the needle 1 and the channel 3. Positioned at this point of juncture is the valve 5. The flushing channel 4 may of course meet the channel 3 at any desired angle, for example 90°, if the design of the portion 2a is amended.

The valve 5, as may be seen from Figures 2 and 3, may be formed of a supporting annulus 8 which holds a membrane 10 under tension. With reference to Figure 2 plane slits are made in the membrane 10. In Figure 3 overlapping portions 7 replace the plane slits 6 whereby the threshold pressures required to open the slits will be different depending on the direction from which the pressure is applied.

The arrangements as just described are particularly useful in oocyte recovery which is a procedure used in both in-vitro fertilisation and gamete intrafallopian transfer techniques. In these techniques the needle 3 is passed by transcutaneous puncture and is directed by ultrasound or laparscopy to an ovarian follicle. The follicle is punctured by the needle in order to aspirate the oocytes via the needle to a collection vessel.

Previously there have been two types of oocyte recovery needles in use: single channel as hereinbefore described, and double channel. In both cases a Heparinised solution is introduced to flush oocytes from the follicles. Although a double channel needle makes aspiration of the freed oocytes more readily achieved, it also means a larger diameter needle which increases trauma during its introduction and also on puncture of the follicle. Further, the application of negative pressure to the aspirating channel of the double channel needle can result in oocytes being lodged in the Heparin channel thereby resisting aspiration. For this reason a single channel needle tends to cause less problems in that its introduction and follicle puncture can be achieved with less trauma. In this arrangement Heparin is expressed through the needle via the flushing channel 4. In this process the collection channel 3 is temporarily obturated by compression, allowing the Heparin solution to pass to the follicles. This may be achieved because Heparin from a hypodermic syringe, for example, can be introduced under pressure down the flushing tube 4, the pressure exceeding the threshold pressure and thereby allowing the Heparin solution to pass into the lumen of the needle 1. When a sufficiency of Heparin solution has passed down the needle 1 the oocytes are withdrawn by aspiration along the collecting channel 3. The negative pressure applied is below the threshold value of the valve membrane 5 and, with the compression removed, the aspirated Heparinised solution bearing oocytes is withdrawn to the collecting vessel. This arrangement prevents the oocytes from passing into the channel 4, avoids bruising them, and prevents Heparinised solution remaining in the channel 4 from flowing into the collection vessel. This is important because it is only possible to correctly aspirate the follicle if the valve 5 is closed. —

Accordingly, the invention provides a pressure actuated valve as hereinbefore described, a membrane for such a valve, and a method for the operation of a presure actuated valve.

**Claims**

1. A single channel needle assembly for the recovery of a biologically active component, which assembly comprises;
a single channel transcutaneous needle (1),
a three-way connector assembly (2) operatively associated with a remote end of said needle;
characterised in that one channel (4) of said three-way assembly (2) is obturated by a valve assembly (5) comprising a resilient membrane (10) provided with at least one slit (6.7.) held under tension across said one channel, said resilient membrane being arranged such that the slit remains closed during normal aspiration.

2. An assembly as claimed in claim 1 characterised in that the membrane is formed with at least two layers with slits (7) out of register but in communication, thereby to form an overlap slit.

3. An assembly according to claim 2 wherein the slits overlap to provide different pressure thresholds at upstream and downstream sides of the membrane.

4. A method for the recovery of a biologically active component, which method comprises;
inserting a single channel needle assembly into the body to a site juxtaposed said component,
introducing a flushing solution into the body via said needle, and aspirating said component via said single channel;
characterised in that the remote end of the needle (1) is operatively associated with a three-way valve assembly (2), said valve assembly being provided in one channel (4) with a resilient membrane (5) provided with at least one slit (6.7.) held under tension across said one channel, said resilient membrane being arranged such that said slit remains closed during normal aspiration, but opens under pressure from said flushing solution.

5. A method according to claim 4 characterised in that the component is a mammalian oocyte, and wherein the flushing solution is introduced by obturating the aspiration channel prior to introduction of the flushing solution under pressure.

6. A method of inducing pregnancy in an infertile mammalian female, which method comprises recovering an oocyte from the follicle by inserting a single channel needle assembly into the ovarian follicle,
introducing a flushing solution into the follicle via said needle, aspirating said component via said single channel, causing said oocytes to be fertilised in vitro and implanting said fertilised oocytes into the uterine endometrium to establish pregnancy, characterised in that the recovery of the oocytes is effected by an arrangement wherein the remote end of the needle (1) is operatively associated with a three-way valve assembly (2), said valve assembly being provided in one channel (4) with a resilient membrane (5) provided with at least one slit (6.7.) held under tension across said one channel, said resilient membrane being arranged such that said slit remains closed during normal aspiration, but opens under pressure from said flushing solution in use.

7. A method according to claim 6 characterised in that the solution is a heparinized solution.

*FIG.1*

4

2a

1

3

5   9   2

*FIG.2*

5

10

6   8

*FIG.3*

5

10

7   8